# EUROPEAN PATENT APPLICATION

(11) **EP 4 009 334 A1**
(43) Date of publication of application: **08.06.2022**
(21) Application number: 20211466.6
(22) Date of filing: 03.12.2020
(51) Int. Cl.: G16H 50/00

(54) **ANGIOGRAPHY DERIVED CORONARY FLOW**

(71) Applicant: Koninklijke Philips N.V., 5656 AG Eindhoven (NL)
(72) Inventor: HAASE, Christian, 5656 AE Eindhoven (NL); GRASS, Michael, 5656 AE Eindhoven (NL)
(74) Representative: Philips Intellectual Property & Standards

(57) **Abstract**

An apparatus and a method for assessing a vasculature is provided in which a time series of diagnostic images is used in combination with at least one boundary parameter associated with said time series to determine a quantitative fluid dynamics parameter indicative of the fluid flow through the vasculature using a trained classifier. By providing both, the time series of diagnostic images and the at least one boundary parameter to the determination, it is ensured that the classifier is provided with consistent data allowing for a more accurate determination of the quantitative fluid dynamics parameter.

## Description

### FIELD OF THE INVENTION

The present invention relates to an apparatus for assessing a vasculature, a corresponding method and a respective computer program. In particular, the present invention relates to an apparatus for assessing a vasculature, such as a coronary vasculature, using quantitative flow values that have been derived based on a classification result and a combination result for a time series of diagnostic images and at least one boundary parameter.

### BACKGROUND OF THE INVENTION

Coronary blood flow measurements are an important tool for the assessment and analysis of coronary artery disease, as they allow improving the understanding of a coronary lesion. More specifically, these measurements allow determining consequences of coronary artery disease, such as the ischemic potential, and also provide treatment guidance for a patient. For this purpose, coronary blood flow measurements may be performed. These measurements shall hereby be performed under resting conditions and under hyperemic conditions in order to determine flow-related properties, such as flow velocity or volumetric flow rate for both states.

Based on these measurements, various flow-related indices can be determined, such as the so-called Coronary Flow Reserve (CFR) or the microvascular resistance. The CFR defines the maximum increase in blood flow through the vasculature above the normal resting volume and may be calculated from the ratio between the hyperemic flow velocity *ν_{H}* and the resting flow velocity *ν_{R}.*

Despite the well-known benefits of these measurements, routine flow measurements have not found their way into clinical practice, due to the added complexity and the lack of robustness of the measurement techniques that are currently available for measuring flow-related parameters.

### SUMMARY OF THE INVENTION

Thus, approaches have been made to avoid the need for measuring flow-related parameters directly and instead allow deriving these properties indirectly from measurements other than flow measurements.

One such approach is to derive the flow-related property values, such as the flow velocity values or microvascular resistance from the contrast agent dynamics as obtained from a time series of diagnostic images, such as X-ray angiography images. While such angiography-derived flow values from contrast agent dynamics could be used to provide a simplified approach for obtaining flow-based diagnostic information, these approaches are typically not very accurate, affected by measurement errors and are generally complex which makes them unsuitable for clinical practice. That is, thus far, no sufficiently accurate, robust and simple method has been developed to be used in clinical practice.

It is therefore an object of the invention to provide an apparatus and a method which allow for an accurate and simple determination of flow-related indices. More specifically, it is an object of the invention to provide an apparatus and a method which allow to indirectly determine those flow-related indices in a more accurate, robust and simple manner than known approaches.

This object is achieved by an apparatus for assessing a vasculature, comprising an input unit configured to receive a time series of diagnostic images of the vasculature and at least one boundary parameter associated with said time series of diagnostic images, a computation unit comprising a trained classifier device, whereby the computation unit is configured to generate a combination result based on the time series of diagnostic images and the at least one boundary parameter and determine, using the trained classifier device, a quantitative fluid dynamics parameter indicative of the fluid flow through the vasculature based on the combination result.

In this context, the term vasculature may be understood as referring to a plurality of vessels of a patient. In some embodiments, the term vasculature may refer to a plurality of vessels of one or more vessel trees. In some embodiments, the vasculature may correspond to a coronary vasculature. In some embodiments, the term vasculature may also refer to sub-branches, such as the LAD or LCX of one vessel tree. In some embodiments, the vasculature may correspond to peripheral or abdominal vasculature or to a neurological vasculature.

Further, the term time series of diagnostic images may particularly be understood as referring to a plurality of diagnostic images that have been acquired over time, more particularly over a certain time span. More specifically, the time series of diagnostic images may comprise a plurality of diagnostic images which allow to visualize the progression of a contrast agent through the vasculature that is visualized in the images. This allows tracking of the contrast agent dynamics through said vasculature, which, in turn, allows deriving information about the fluid flow properties through the vasculature in question. The diagnostic images may hereby be acquired by any medical imaging modality capable of visualizing contrast agent in the vasculature. One particular imaging method which allows acquiring the diagnostic images is X-ray imaging.

Each one of the diagnostic images of the time series may hereby represent the state of the contrast agent progression at a particular point in time. To this end, the term point in time shall be understood as referring to a certain point in measurement time relative to the moment of injection of the contrast agent. In other words, for each measurement, the time is measured starting at the moment at which the contrast agent is injected. In some embodiments, the acquisition of the diagnostic images may particularly be initiated upon injection of the contrast agent. In some embodiments, the acquisition of the diagnostic images may, alternatively or additionally, be initiated at a certain point after the contrast agent injection has started.

The term boundary parameter is to be understood as referring to a parameter that defines a boundary condition for the particular measurement modality and measurement parameters with which the time series of diagnostic images has been acquired. Hereby, at least one boundary parameter may be used. In some embodiments, a plurality of boundary parameters may be used.

In some embodiments, the boundary parameter may correspond to a system parameter, i.e. a boundary condition that is specified by the system, such as the frame rate for the diagnostic images, the projection angle, the projection resolution of the diagnostic images or the like.

In some embodiments, the boundary parameter may, alternatively or additionally, correspond to a measurement boundary parameter, i.e. a boundary condition that is specified by the particular measurement, such as the contrast agent injection rate, the contrast agent volume, the contrast agent dilution, the injection pressure, the injection timing or the like.

The fact that the at least one boundary parameter is associated with said time series of diagnostic images may be understood as referring to the fact that the at least one boundary parameter provides for a boundary condition for the particular measurement with which the particular time series of diagnostic images was acquired. That is, the at least one boundary parameter is relevant for that particular time series.

The term classifier device may be understood as referring to any device capable of employing machine and/or deep learning algorithms. In some embodiments, the classifier device may particularly correspond to a neural network comprising a plurality of neural nodes. In some embodiments, the classifier device may particularly refer to a 2.5 D encoder network architecture.

The classifier device may particularly be and/or comprise a trained classifier device. This is to be understood as referring to the understanding that the classifier device has previously been trained with a respective ground truth for the classification to be performed. Hereby, said training may be based on a training dataset for said ground truth.

In some embodiments, the training dataset may have been derived on the basis of measurement datasets. In some embodiments, the training dataset may, alternatively or additionally, have been derived on the basis of a simulation and/or modelling which output may then be used as the training dataset. In some embodiments, the training dataset may correspond to a virtual dataset that has been generated by a different manner than a simulation and/or modelling.

The term combination result may be understood as referring to the result of a processing of the time series of diagnostic images based on the at least one boundary parameter. In some embodiments, the combination result may particularly correspond to the result of adjusting the properties of the diagnostic images in the time series using the at least one boundary parameter, such as normalizing the frame rate and/or the resolution of the diagnostic images, adjusting the image contrast of the diagnostic images, adjusting a sequence length of the time series of diagnostic images, leaving out particular projection angles in the time series of diagnostic images and/or preferring particular projection angles in the time series of diagnostic images or the like.

The trained classifier device may particularly be used to determine a parameter indicative of the fluid flow through the vasculature. This determination may be based on the combination result. In this context, the terms using and based on have to be interpreted broadly.

More particularly, the phrase using the trained classifier device means that the trained classifier device performs one or more steps in the processing of the time series of diagnostic images and the at least one boundary parameter. In some embodiments, the classifier device may particularly perform a classification step. In some embodiments, this classification step may include classifying the time series of diagnostic images prior to the time series of diagnostic images being processed to generate the combination result. In some embodiments, the classification step may include classifying the combination result prior to processing the combination result in order to obtain the quantitative fluid dynamics parameter indicative of the fluid flow through the vasculature. In some embodiments, the classifier device may, alternative or additionally, be employed to determine the quantitative fluid dynamics parameter itself.

Hereby, the quantitative fluid dynamics parameter may particularly be determined based on the combination result. This may particularly be interpreted as meaning that, in order to determine the quantitative fluid dynamics parameter, the time series of diagnostic images is first processed, in particular adjusted, based on the at least one boundary parameter to generate the combination result. The thus generated combination result is then processed to output the quantitative fluid dynamics parameter indicative of the fluid flow through the vasculature.

The term quantitative fluid dynamics parameter shall hereby be understood as referring to a quantitative parameter about the fluid flow properties through the vasculature. The quantitative fluid dynamics parameter may take a particular value for different time series. In some embodiments, the quantitative fluid dynamics parameter may for particularly be a hemodynamic index, such as coronary flow reserve (CFR) or fractional flow reserve (FFR) or the like. In some embodiments, other flow parameters which allow to specify the properties of the fluid flow, particularly the blood flow, through the vasculature may be foreseen.

Hereby, the variety of fluid dynamics parameters that may be provided is large and particularly depends on the training of the trained classifier device. That is, it, the trained classifier device may be trained such as to provide a variety of different classification results. In some embodiments, these classification results may particularly comprise qualitative fluid flow values for the different vessels in the vasculature which may then be processed to obtain the fluid flow ratio between the different vessels. In some embodiments, the classification results may provide fluid flow values for different time series of diagnostic images, e.g. one time series representative of a resting condition and one time series representative of a hyperemic condition of a patient, which may then be used to determine a fluid flow ratio between these different time series, such as to give a value such as the CFR.

In some embodiments, the trained classifier device may be used to track the contrast agent front progression over time and use this progression to determine quantitative flow values by further using information such as the frame rate and/or the pixel spacing for the particular diagnostic images. In some embodiments, the contrast agent front progression may particularly be tracked by obtaining vessel segmentations from the diagnostic images and/or using percentage filling values for the contrast agent filling in particular vessels of the vasculature.

In some embodiments, the time series of diagnostic images may be used to track contrast agent outflow and/or contrast agent inflow for the vessels in the vasculature. Based on this tracking, fluid flow values may be obtained and then averaged with one another. In some embodiments, particularly fluid flow values from different vessels in the vasculature may be adjusted in order to obtain consistency throughout the vasculature. As an example, by means of the adjustment it may be ensured that for two branches from a parent branch, both branches together have the same combined volumetric flow as the respective parent branch.

In some embodiments, the trained classifier device may use time series of diagnostic images showing a consistent ostial backflow of contrast agent in the vasculature. The contrast agent filled vessels may then be segmented. Hereby, the contrast agent value of the segmented vessel over time may particularly be equal to the volumetric flow since no additional blood could enter the coronary tree and it is filled only with contrast agent. This allows to more precisely obtain information about the fluid flow dynamics through the vasculature.

In some embodiments, the trained classifier device may also be used to identify a transition between the presence of backflow of contrast agent and the absence thereof. Based on this, the corresponding contrast agent injection rate may be assumed to correspond to the volumetric flow rate at the ostium, thereby giving a further indication of the fluid dynamic properties in the vasculature.

In some embodiments, the absence of backflow in a classification result of the trained classifier device in combination with a segmentation of one or more vessels in the vasculature that has been performed based on the diagnostic images, and a respective one-dimensional reformatting of a vessel centerline, may allow deriving the flow speed, in particular the blood flow speed, based on a peak to peak distance of the inner vessel contrast modulation. That is, since any backflow is absent, it can be assumed that the contrast agent is mixed with a variable amount of blood over a heart cycle, thereby resulting in an indication of the flow speed.

Further possibilities of deriving fluid dynamic parameters and/or fluid dynamic properties based on the classification are also foreseen by the invention and are immediately evident to the skilled person.

In accordance with the invention, a trained classifier is used to determine the quantitative fluid dynamics parameter. The use of a trained classifier, such as a neural network, enhances accuracy, reproducibility, and ease of application. Hereby, the trained classifier may particularly be trained to use a large number of clinical (i.e. measured) as well as simulated data. A particular benefit of such a trained classifier resides in the fact that said trained classifier continues learning even during use. This allows increasing accuracy even more.

Hereby, the particular insight on which the present solution is based is such that a trained classifier is only capable of obtaining and maintaining the above-mentioned reliability and accuracy brought forward by trained classifiers if the datasets input into the trained classifier have a consistent structure. The solution takes into account the realization that this consistence is not naturally present in typical clinical/measured data.

As an example, when using random diagnostic images having random frame rates, image resolution, image sizes and the like as clinical/measured data, the continued training of the trained classifier may possibly become less accurate, due to the inconsistency in the data obtained. In order to avoid this, an adjustment on the basis of the boundary parameters is performed for the data.

As a specific example for sake of further explanation, the obtaining of a time series of diagnostic images using dual-energy X-ray angiography shall be mentioned. Here, the adjustment may comprise providing information about a specific spectral decomposition in the diagnostic images as the at one boundary parameter associated with the time series of diagnostic images. This may allow the trained classifier device to derive a quantitative contrast agent volume in some vessels or regions of the vasculature. Without, however, providing the spectral decomposition information as a boundary parameter, the derivation of such quantitative values would not be possible.

Hence, an apparatus is provided in which the trained classifier is able to learn the ground truth based on consistent data.

The apparatus therefore enables a more accurate, robust and simple determination of a quantitative fluid dynamics parameter by tracking contrast agent progression through said vasculature using an appropriate imaging modality and deriving the flow properties on the basis of said progression tracking using a consistently trained classifier device.

In some embodiments, the computation unit may further comprise a processing unit, wherein the trained classifier device may be configured to receive the time series of diagnostic images, classify the time series of diagnostic images based on a trained ground truth to generate a classification result and provide the classification result to the processing unit, wherein the processing unit may be configured to receive the classification result, generate the combination result based on the classification result and the at least one boundary parameter and determine the quantitative fluid dynamics parameter based on the combination result.

In some embodiments, the computation unit may comprise a processing unit and the trained classifier device. In some embodiments, the trained classifier device may hereby be configured to receive the time series of diagnostic images directly, i.e. prior to generation of the combination result. Hereby, the trained classifier device may be configured to classify the time series of diagnostic images. For this purpose, the classifier device may initially be trained with a ground truth for the classification of the time series of diagnostic images. In some embodiments, the ground truth may particularly allow classifying the diagnostic images based on the contrast agent distribution - and, hence, the contrast agent progression - visible therein. The contrast agent distribution may hereby particularly provide an indication about the fluid flow properties through the vasculature.

The trained classifier device may particularly receive the time series of diagnostic images and classify them based on the trained ground truth. This allows generating a classification result. In this context, the term classification result may particularly be understood as specifying the result of the classification of the diagnostic images in view of the ground truth, i.e. in relation to the contrast agent distribution. The classification result thus may particularly comprise a plurality of classified diagnostic images.

Subsequently, the trained classifier device may provide the classification result to the processing unit. The processing unit may receive the classification result from the trained classifier device and process the classification result in order to generate a combination result. Hereby, the processing may particularly refer to adjusting the plurality of diagnostic images on the basis of the at least one boundary parameter. The adjusting may hereby comprise a normalization of a frame rate and/or an image resolution, an adjustment of the contrast of the diagnostic images and/or a deletion and/or preference of particular projection angles or the like as described above.

The processing unit may thus generate the combination result based on the classification result and the at least one boundary parameter. Subsequently, the processing unit may use the combination result to determine the quantitative fluid dynamics parameter.

That is, in some embodiments, the classification may take place prior to the adjustment of the diagnostic images using the at least one boundary parameter. The thus generated combination result may then be used to determine a quantitative fluid dynamics parameter which allows to determine the flow properties through the vasculature under investigation.

In some embodiments, the classification of the time series of diagnostic images may be used to obtain an abstract definition of the flow properties through the vasculature. Such an abstract definition may, for example, comprise a number of diagnostic images (i.e. a number of frames) that are obtained until complete filling of a particular vessel or a plurality of vessels in the vasculature by the contrast agent is achieved.

In some embodiments, the classification of the time series of diagnostic images may, alternatively or additionally, give an area classification for the diagnostic images in which particular image areas corresponding to a specific part of the vasculature are extracted and/or registered. As an example, such an extraction and/or registration may give a consistent one-dimensional reformatting of the centerline of the left anterior descending artery (LAD) through all motion states and all contrast agent filling states visualized in the time series of diagnostic images.

In some embodiments, the classification may, alternatively or additionally, give percentages of contrast agent filling states for a plurality of different vessels in the vasculature. In some embodiments, a plurality of these kinds of classifications may be combined into one or more vectors.

In some embodiments, the classification of the diagnostic images may, alternatively or additionally, give a contrast agent dilution. This contrast agent dilution may be used as a flow property for subsequent determination of the quantitative fluid dynamics parameter. In these embodiments, the trained classifier may, upon classifying, segment one or more vessel outlines of the vasculature visualized in the diagnostic images, while at the same time select only injections with or without contrast agent backflow, e.g. from the ostium. A presence or absence of contrast agent backflow may be used as a measure to determine if the chosen contrast injection rate was adequate. This allows to either avoid or achieve contrast agent blood mixing in the arteries.

In some embodiments, the classification may particularly be performed such as to determine a specific fluid dynamics parameter that is deemed particularly desired for that particular patient.

As an example, when the time series of diagnostic images is obtained using X-ray angiography and shall be used to determine the coronary flow reserve (CFR) as the quantitative fluid dynamics parameter, an abstract measure of the contrast agent flow speed and/or contrast agent flow progression through the vasculature can be obtained for a specific X-ray angiogram. Hereby, factors such as frame rate, image resolution or the like may be ignored. This is the case since in the determination of the CFR, it is sufficient to regard a relative change of the abstract fluid flow speed and/or fluid flow progression in two identical angiograms which have been obtained under hyperemia and under rest, respectively.

In some embodiments, the computation unit may further comprise a processing unit, wherein the processing unit may be configured to generate the combination result based on the time series of diagnostic images and the at least one boundary parameter and provide the combination result to the trained classifier device. Hereby, the trained classifier device may be configured to receive the combination result, classify the combination result based on a trained ground truth to generate a classification result, and provide the classification result to the processing unit. The processing unit may further be configured to receive the classification result based on the combination result, and determine the quantitative fluid dynamics parameter based on the classification result.

In some embodiments, the computation unit may also comprise a processing unit and the trained classifier device, whereby the time series of diagnostic images may be adjusted, by the processing unit, based on the at least one boundary parameter prior to being provided to the trained classifier device. Hereby, the processing unit may particularly be configured to receive the time series of diagnostic images and process the time series of diagnostic images based on the at least one boundary parameter. This processing may particularly comprise an adjusting of the diagnostic images in the time series as discussed above. The processing unit may thus generate a combination result comprising a plurality of diagnostic images of the time series that have been adjusted based on the at least one boundary parameter in order to provide a consistent dataset.

The combination result may then be provided to the trained classifier device. The trained classifier device may be configured to receive the combination result and classify the combination result. For this purpose, the classifier device may be trained with a respective ground truth. In some embodiments, the ground truth may particularly allow classifying the adjusted diagnostic images in the combination result based on the contrast agent progression indicated therein as said contrast agent distribution may provide an indication about the fluid flow properties through the vasculature.

The trained classifier device may thus classify the combination result comprising the plurality of diagnostic images previously adjusted based on the ground truth to generate a respective classification result. The trained classifier device may then provide the classification result to the processing unit again. The processing unit may use the classification result based on the combination result in order to determine the quantitative fluid dynamics parameter for the vasculature.

In accordance with this embodiment, the time series of diagnostic images is adjusted based on the at least one boundary parameter prior to being provided to the trained classifier device. This allows for more consistent datasets being provided as input of the classification, which thereby improves the accuracy of the subsequent classification results provided.

In some embodiments, the trained classifier device may be trained with a ground truth for the quantitative fluid dynamics parameter, wherein the trained classifier device may be trained using a virtual time series of diagnostic images indicative of a contrast agent dynamic through the vasculature. In some embodiments, the virtual time series of diagnostic images may be generated by defining at least one virtual vessel tree, defining a virtual contrast agent injection rate, and modelling the flow speed through the least one vessel tree based on a fluid dynamics model.

The classifier device has to be trained. Hereby, the training may be performed using a respective training dataset. Said training dataset may particularly be similar to the dataset actually acquired in the use case on the basis of which the assessment shall be performed. Hereby, the training dataset may particularly correspond to the dataset provided to the trained classifier device in order to be classified.

In some embodiments, this may mean that the training dataset shall correspond to a dataset similar to the time series of diagnostic images. As an example, if the use case encompasses classifying a time series of diagnostic images that have been acquired using X-ray angiography prior to determining the combination result, the training dataset may particularly correspond to a time series of diagnostic X-ray angiography images. In some embodiments, the combination result is first generated and then provided for classification. In this case, the training dataset may particularly correspond to a dataset indicative of the combination result. As an example, if the use case encompasses classifying a combination result comprising a time series of diagnostic X-ray angiography images that have been adjusted based on the projection angle and the image resolution as boundary parameters, the training dataset may also comprise one or more of diagnostic X-ray images of a time series that have been adjusted based on the projection angle and image resolution.

In some embodiments, the training dataset may particularly be generated based on a virtual time series of diagnostic images. To that end, the term virtual is to be interpreted such as to refer to a time series of diagnostic images that has not actually been acquired, but has been generated based on a computer model.

For that purpose, a virtual vasculature, such as a virtual coronary tree, may be specified. Subsequently, the injection and flow of the contrast agent may be defined by defining a virtual contrast agent injection rate and a virtual contrast agent flow speed in the tree. Hereby, variable flow speeds through the vasculature may be defined by means of a respective fluid dynamics model, such as a lumped parameter model. Hereby, the flow speeds may be varied by varying the microvascular resistance of the vasculature as a boundary condition.

In this context, the term fluid dynamics model may particularly refer to a model of the blood flow through the vessel of interest. This fluid dynamics model is generated by simulating the interaction of the blood with the vessel wall. Hereby, the fluid dynamics model may particularly refer to a lumped parameter model. To that end, the term lumped parameter model may particularly refer to a model in which the fluid dynamics of the vessels are approximated by a topology of discrete entities. Hereby, the vasculature may be represented by a topology of resistor elements each having a particular resistance with the representation of the vasculature being terminated by respective ground elements. These lumped parameter models reduce the number of dimensions compared to other approaches such as Navier-Stokes or the like. The employing of such a lumped parameter model is described, for example, in international application WO 2016/087396. As may be appreciated from this application, the use of a lumped parameter model for fluid flow modelling allows obtaining information about the fluid flow through a vasculature based on an imaging approach.

The virtual vasculature, in particular the virtual vessel trees, are additionally combined with a motion model, such as to take account of the motion of the vessels. Using this, two separate datasets may particularly be generated as training datasets.

The first training dataset may particularly be obtained by forward projecting the vasculature in motion onto an empty clinical background and simulating a time series of diagnostic images for a plurality of different flow speeds, injection times. Contrast agent concentration and imaging frames over a given time span. Further, the background and the structure of vasculature is varied in order to obtain a widely suitable training dataset.

The second training dataset may be generated by also forward projecting the diagnostic images but considering these objections at one particular contrast injection time, contrast agent concentration and imaging frame, whereby the flow speeds in the coronary trees remain the same as before.

The training itself may then be performed according to known methods for training the classifier device, such as back propagation, an Adam optimizer with batch normalization or the like.

In some embodiments, in order to train the classifier device, the second training dataset may be provided to the classifier device and providing, as a ground truth output, data indicative of an average fluid flow velocity as obtained from the fluid dynamics model. The classifier device may then be trained for multiple rounds. Subsequently, the trained classifier device may be applied to the first training dataset in order to provide a plurality of quantitative fluid dynamics parameter values.

These quantitative fluid dynamics parameter values and the first training dataset may then be used as input to a linear regression. The result of said linear regression may particularly correspond to a correction factor to be used in the classification.

In some embodiments, the combination result may be generated by using the at least one boundary parameter associated with said time series of diagnostic images to perform an adjustment of the time series of diagnostic images. In some embodiments, the adjustment may comprise one or more of: a normalization of a frame rate, an adjustment of an image contrast, a normalization of an image resolution, an adjustment of a sequence length, a selection of projection angles or the like.

In some embodiments, the combination result may comprise a plurality of diagnostic images from the time series of diagnostic images that has been adjusted based on the at least one boundary parameter. Hereby, the adjustment may particularly correspond to an adjustment that enhances comparability of the different time series of diagnostic images, in particular in relation to the training dataset.

Hereby, in some embodiments, the adjustment may particularly comprise a normalization of the frame rate for the diagnostic images, i.e. a normalization as to the number of diagnostic images acquired in a particular time span. This allows using one particular timing for all time series of diagnostic images and, thus, allows removing possible inaccuracies due to different frame rates and, thus, different timing conditions between the different datasets.

Alternatively or additionally, the adjusting may also comprise an adjustment of the image contrast and/or a normalization of the image resolution of the diagnostic images, such as to achieve a particular specified image contrast and/or image resolution for the diagnostic images.

In some embodiments, the adjusting may, alternatively or additionally, comprise an adjustment of a sequence length of the time series of diagnostic images, i.e. may equalize the number of diagnostic images in the time series that is obtained in one particular sequence.

Alternatively or additionally, the adjusting may further comprise the selection of one or more projection angles for further processing. That is, in some embodiments, diagnostic images having one or more particular projection angles may be removed from the time series of diagnostic images when generating the combination result. In some embodiments, diagnostic images having one or more projection specific projection angles may be preferred in the time series of diagnostic images. This allows to exclude/include particularly relevant data and, thereby, improve the accuracy of the assessment.

In some embodiments, the at least one boundary parameter may comprise at least one system parameter and/or at least one measurement boundary parameter. In some embodiments the at least one boundary parameter may comprise one or more of: a frame rate, a projection angle, a projection resolution, a contrast agent injection rate, a contrast agent volume, a contrast agent dilution, an injection pressure, an injection timing.

In some embodiments, the at least one boundary parameter may comprise at least one system parameter. Hereby, the term system parameter is to be understood as referring to a boundary parameter which boundary is specified by the system properties. As examples for such a system parameter, one or more of a frame rate, a projection angle, a projection resolution or the like may be mentioned.

Alternatively or additionally, the at least one boundary parameter may comprise at least one measurement boundary parameter. The term measurement boundary parameter may particularly be understood as referring to a boundary parameter which boundary is specified by the measurement settings. As examples for such measurement boundary parameters a contrast agent injection rate, a contrast agent volume, a contrast agent dilution, an injection pressure, an injection timing or the like shall be mentioned.

In some embodiments, the computation unit may comprise a processing unit and the processing unit may comprise a second trained classifier device.

As discussed herein above, in some embodiments, the computation unit comprises a trained classifier device for classifying and a processing unit for adjusting and determining the quantitative fluid dynamics parameter. In some embodiments, the processing unit for adjusting and determining may also comprise and/or be implemented as a classifier device. In this case also, the second classifier device may be trained with a respective ground truth prior to being used.

Hereby, said training may also be based on a training dataset that may have been derived based on measurement datasets. In some embodiments, the training dataset may, alternatively or additionally, have been derived on the basis of a simulation and/or modelling which output may then be used as the training dataset. In some embodiments, the training dataset may correspond to a virtual dataset that has been generated by a different manner than a simulation and/or modelling.

In some embodiments, the virtual dataset may particularly be generated such as to comprise a subset of data that is as inconsistent and diverse as typical clinical data. Further, the virtual dataset may be generated to comprise a subset of data associated with the inconsistent/diverse data that provides for a consistent counterpart thereto. To put differently: a simulated case may be generated for which both an inconsistent data subset as well as a consistent data subset is provided. Hereby, the inconsistent subset of the virtual dataset may particularly comprise random values for the image resolution, the frame rate, the contrast or the like. The consistent subset may comprise normalized information. Based on these subsets, the classifier device may then be trained how to interpolate the consistent subset with the inconsistent subset, such as to obtain consistent classification results.

In some embodiments, the trained classifier device may, for this purpose, comprise a Generative Adversarial Network (GAN). Using a GAN has the benefit that the trained classifier device comprises two networks that are trained simultaneously, whereby the first network is learning, during training, how output may be provided based on the ground truth and the second network is learning, during training, to classify if the output is actually a network output or the ground truth. Hereby, the improvement of the second network results in a simultaneous improvement of the first network. It shall be understood that the trained classifier device may also comprise other kinds of networks and/or classifiers.

According to another aspect of the invention, a method for assessing a vasculature is provided, the method comprising the steps of receiving a time series of diagnostic images of the vasculature, receiving at least one boundary parameter associated with said time series of diagnostic images, generating a combination result based on the time series of diagnostic images and the at least one boundary parameter, and determining, using a trained classifier device, a quantitative fluid dynamics parameter indicative of the fluid flow through the vasculature based on the combination result.

In some embodiments, the method may further comprise generating, by the trained classifier device, a classification result by receiving the time series of diagnostic images and classifying the time series of diagnostic images based on a trained ground truth, generating the combination result based on the classification result and the at least one boundary parameter, and determining the quantitative fluid dynamics parameter based on the combination result.

In some embodiments, the method may further comprise generating the combination result based on the time series of diagnostic images and the at least one boundary parameter, classifying, by the trained classifier device, the combination result based on a trained ground truth to generate the classification result, and determining the quantitative fluid dynamics parameter based on the classification result.

According to yet another aspect, a computer program is provided, the computer program for controlling an apparatus as specified herein above, when executed by a processing device, is adapted to perform the method as specified herein above. In an even further aspect, a computer-readable medium having stored thereon the computer program is provided.

It shall be understood that the apparatus of claim 1, the method of claim 11, the computer program according to 14, and the computer readable medium of claim 15, have similar and/or identical preferred embodiments, in particular, as defined in the dependent claims.

It shall be understood that a preferred embodiment of the present invention can also be any combination of the dependent claims or above embodiments with the respective independent claim.

These and other aspects of the invention will be apparent from and elucidated with reference to the embodiments described hereinafter.

### BRIEF DESCRIPTION OF THE DRAWINGS

In the following drawings:
Fig. 1 schematically illustrates an apparatus for assessing a vasculature according to a first exemplary embodiment,
Fig. 2 represents a flow chart for a method for assessing a vasculature according to the first exemplary embodiment,
Fig. 3 schematically illustrates an apparatus for assessing a vasculature according to a second exemplary embodiment,
Fig. 4 represents a flow chart for a method for assessing a vasculature according to the second exemplary embodiment,
Fig. 5 represents a flow chart for a method for training a classifier device based on a virtual training dataset in accordance with the invention,
Fig. 6 represents a flow chart for a method for classifying an input dataset in accordance with the invention, and
Fig. 7 schematically illustrates an exemplary embodiment for a neural network that may be used as a classifier device according to the invention.

### DETAILED DESCRIPTION OF EMBODIMENTS

The illustration in the drawings is schematically. In different drawings, similar or identical elements are provided with the same reference numerals.

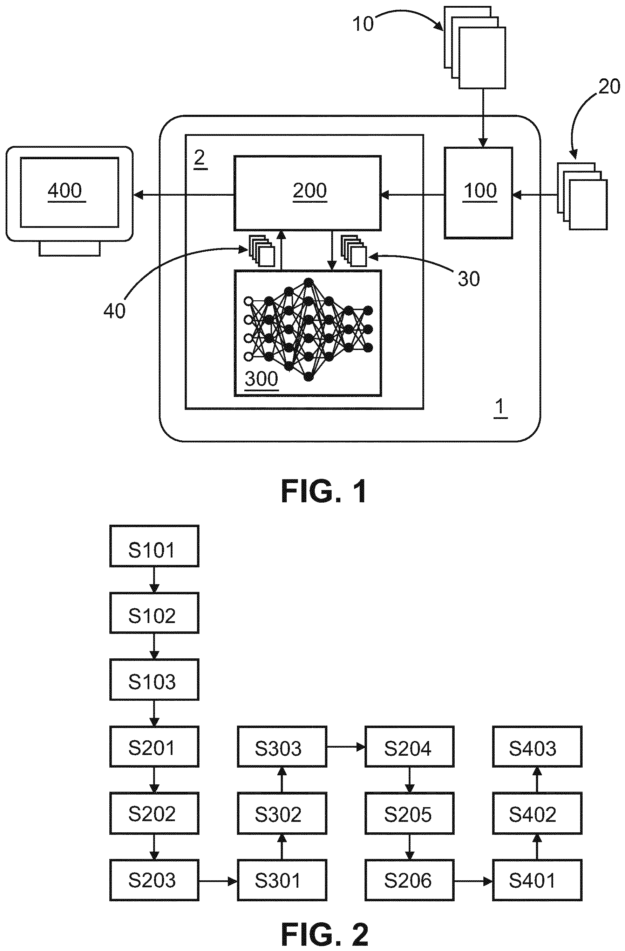

Fig. 1 represents schematically a first exemplary embodiment of an apparatus 1 for assessing a vasculature, in particular a coronary vasculature, based on at least one quantitative fluid dynamics parameter, such as a coronary flow reserve, that has been derived on the basis of a time series of diagnostic images and a set of associated boundary parameters.

The apparatus 1 comprises an input unit 100 and a computation unit 2. In the specific embodiment of Fig. 1, the computation unit 2 comprises a processing unit 200 and a trained classifier device 300. The trained classifier device 300 is, in the exemplary embodiment according to Fig. 1, implemented as a neural network comprising a plurality of nodes that are interconnected with one another, such that information input to the neural network can be communicated amongst the individual nodes.

The apparatus 1 may further comprise or be communicatively connected to a display device 400 which is configured to generate a graphical representation of the results of the assessment performed by apparatus 1 and present the graphical representation to a user, such as a physician, e.g. for potential further analysis and/or therapy planning.

Input unit 100 is configured to receive a time series of diagnostic images 10. In the specific exemplary embodiment according to Fig. 1, these diagnostic images have been obtained using X-ray angiography of a coronary vasculature upon contrast agent injection. As such, the diagnostic images in the time series of diagnostic images are indicative of the progression of the contrast agent through the vasculature over time. This is the case since, by using X-ray angiography, the contrast agent is visible in the diagnostic images and, accordingly, it can be tracked, where in the vasculature the contrast agent has already progressed and where no contrast agent may be found. Since the time series of diagnostic images allows tracking the progression of the contrast agent through the vasculature, they may be used to derive information on the fluid flow properties through the vasculature.

Input unit 100 is further configured to receive a dataset 20 specifying at least one boundary parameter. In the exemplary embodiment of Fig. 1, the dataset 20 specifies multiple boundary parameters comprising both, system parameters and measurement boundary parameters. More particularly, in the specific embodiment according to Fig. 2, the input unit receives a dataset 20 comprising an indication about the frame rate, the projection angle and the projection resolution for the time series of the diagnostic images received at the input unit as system parameters as well as a contrast agent injection rate, a contrast agent volume, a contrast agent dilution, and injection pressure and an injection timing for the time series of the diagnostic images as measurement boundary parameters. Accordingly, the system and measurement boundary parameters received are associated with the time series of diagnostic images.

It shall be noted that, although the dataset 20 comprising the at least one boundary parameter comprises all these different parameters, also only a subset thereof may be considered to assess the vasculature.

The input unit 100 then provides the time series of diagnostic images 10 and the dataset 20 specifying the associated set of boundary parameters to computation unit 2. In the exemplary embodiment according to Fig. 1, the time series of diagnostic images 10 may particularly be provided to processing unit 200. Processing unit 200 receives the time series of diagnostic images 10 and the dataset 20 and processes them in order to generate a combination result based thereon.

In the specific exemplary embodiment of Fig. 1, this processing, by the processing unit 200, particularly encompasses adjusting the diagnostic images in the time series using the dataset 20 of boundary parameters.

In order to perform such an adjustment, the processing unit 200, in the specific embodiment according to Fig. 1, normalizes the frame rate of the time series of diagnostic images. This normalization may be performed by leaving out particular frames of the time series of diagnostic images. Alternatively or additionally, the normalization may be performed by interpolating individual frames of the time series of diagnostic images. The normalization may hereby particularly be performed using respective system parameters as boundary parameters, such as the frame rate of the time series of diagnostic images.

In the specific embodiment according to Fig. 1, the adjustment further comprises an image contrast adjustment of the diagnostic images in the time series of diagnostic images. Such image contrast adjustment may particularly be performed, by the processing unit 200, based on measurement boundary parameters as boundary parameters, in particular measurement boundary parameters relating to the contrast agent properties. These measurement boundary parameters may hereby particularly include parameters such as the concentration of the contrast agent upon injection, the contrast agent volume and/or the contrast agent injection rate.

Further, in the specific embodiment according to Fig. 1, the adjustment comprises a normalizing of the image resolution based on respective system parameters as boundary parameters, such as the projection resolution. Subsequently, the image sequence length may be adjusted to the contrast timing based on one or more measurement parameters as boundary parameters, such as the contrast agent volume or the like. This processing results in the most meaningful images being passed to the trained classifier device.

In the specific embodiment of Fig. 1, the adjustment of the time series of diagnostic images further comprises selecting particular projection angles to generate a combined stack of projection images from the diagnostic images. Hereby, some projection angles may be excluded, while others may be more preferred. This adjustment once more may make use of respective system parameters and/or measurement boundary parameters, such as the projection angle and/or a projection resolution or the like.

In the exemplary embodiment according to Fig. 1, the processing unit 200 thus uses the dataset 20 indicative of the boundary parameters to adjust the time series of diagnostic images 10. In doing so, the processing unit 200 generates a combination result 30 based on the time series of diagnostic images 10 and the dataset 20. The processing unit 200 then provides the combination result 30 to the trained classifier device 300.

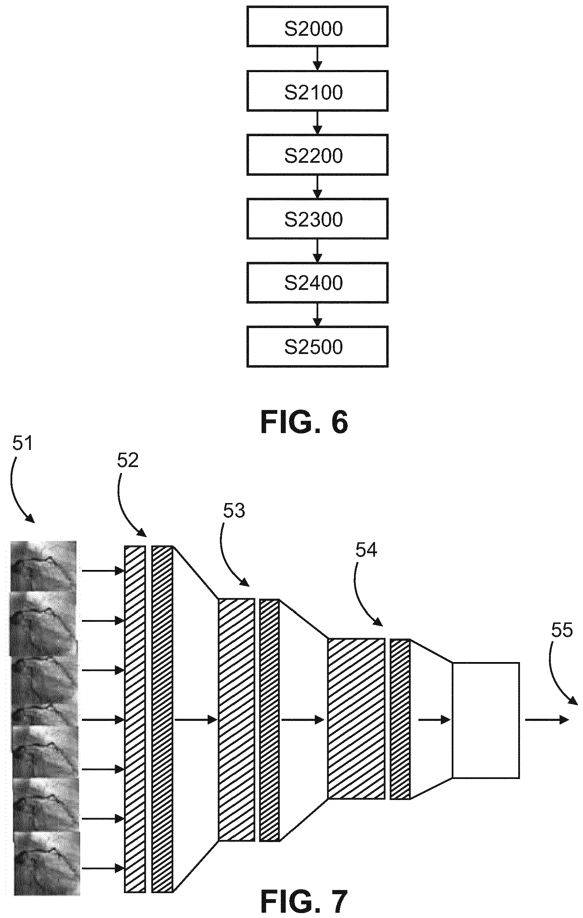

In the exemplary embodiment according to Fig. 1, the trained classifier device 300 corresponds to a neural network, in particular a 2.5D encoder network architecture. The trained classifier device 300 has been trained with a ground truth for classification of the combination result 30 as explained further below with reference to Fig. 6.

The trained classifier device 300 receives the combination result 30 and classifies the combination result 30 based on the trained ground truth to generate a classification result 40. The trained classifier device 300 then outputs the classification result 40 and provides said classification result 40 to the processing unit 200 for further processing.

The processing unit 200 receives the classification result 40 which has been generated based on the combination result 30. In the specific exemplary embodiment according to Fig. 1, the processing unit 200 determines, based on the classification result, a quantitative fluid dynamics parameter for the vasculature that has been shown in the time series of diagnostic images.

In some embodiments, the classification result may particularly provide the quantitative fluid dynamics parameter directly. In these embodiments, the trained classifier device may particularly use the already normalized input to the trained classifier device to predict said quantitative fluid dynamics parameter, such as, for example, a quantitative flow velocity value measured in mm/s.

In some embodiments, the trained classifier may not provide the quantitative fluid dynamics parameter directly, but rather further processing on the classification result is done. As an example, when determining the coronary flow reserve, a first classification result may be provided representative of the patient under rest and a second classification result may be provided representative of the patient under hyperemia. The ratio of these classification results may then be used to determine a CFR value.

In some embodiments, densitometry may be used. In these embodiments, the classification result may comprise an indication about a contrast agent volume in one or more particular vessels of interest in the vasculature. Hereby, the sum of all contrast agent volumes in each of the vessels may, for example, be compared to the amount of contrast agent injected. This may then be normalized using the contrast agent dilution. In case the comparison shows that the summed up amount of the contrast agent in the entire vasculature is smaller than the amount of contrast agent injected, a volumetric flow rate and/or a relative flow rate may be calculated for particular different vessels in the vasculature. Alternatively or additionally, the determination may allow to derive the flow speed of the fluid flow through a particular vessel in the vasculature by using an approximation for the cross sectional area of said vessel. Further possibilities of deriving quantitative fluid dynamics parameters are also foreseen.

An information about the thus determined quantitative fluid dynamics parameter is then provided to display unit 400. Display unit 400 receives the information about the quantitative fluid dynamics parameter and generates a graphical representation thereof. The display unit 400 then displays the graphical representation of the information about the quantitative fluid dynamics parameter on a respective display device for a user to visually acknowledge the information. In some embodiments, the graphical representation provided to the user may further comprise a graphical presentation of the vasculature represented in the time series of diagnostic images. In some embodiments, the graphical representation may comprise a graphical representation of one or more selected diagnostic images from the time series. In some embodiments, the graphical representation may comprise further information, such as information related to the boundary parameters used for that particular dataset.

Fig. 2 schematically represents a method for assessing a vasculature to be performed by the apparatus 1 according to the first exemplary embodiment. It is noted that the flow chart of the method according to Fig. 2 is to be understood as an exemplary embodiment and that the invention is not limited to this exemplary embodiment.

In the exemplary embodiment according to Fig. 2, input unit 100, in step S101, receives a time series of diagnostic images 10 upon contrast agent injection. These diagnostic images may, for example, have been acquired using X-ray angiography, and may, as an example, represent a coronary vasculature.

In the exemplary embodiment of Fig. 2, the diagnostic images in the time series of diagnostic images may particularly be considered as being indicative of the progression of the contrast agent through the coronary vasculature over time. This allows drawing conclusions with respect to the fluid flow properties inside the vasculature.

In step S102, input unit 100 further receives a dataset 20 specifying at least one boundary parameter. The dataset 20 is associated with the time series in that it specifies one or more boundary parameters that relate to the acquisition of the time series of diagnostic images. Specifically, in the exemplary embodiment of Fig. 2, the dataset 20 specifies a plurality of boundary parameters, in particular system parameters, such as frame rate, projection angle, projection resolution or the like and measurement boundary parameter, such as a contrast agent injection rate, a contrast agent volume, a contrast agent dilution, and injection pressure, an injection timing for the time series of the diagnostic images or the like.

In step S103, the input unit 100 then provides the time series of diagnostic images 10 and the dataset 20 specifying the associated set of boundary parameters to processing unit 200 of computation unit 2.

In step S201, processing unit 200 receives the time series of diagnostic images 10 and the dataset 20 and, in step S202, adjusts the diagnostic images in the time series 10 using the dataset 20 of boundary parameters. In the specific embodiment of Fig. 2, this adjustment comprises normalizing the frame rate of the time series of diagnostic images based on the system parameters by leaving out particular frames of the time series of diagnostic images and/or by interpolating individual frames of the time series of diagnostic images. The adjustment may further comprise an image contrast adjustment of the diagnostic images in the time series of diagnostic images based on measurement boundary parameters. Such image contrast adjustment may particularly be performed, by the processing unit 200, based on measurement boundary parameters, in particular measurement boundary parameters relating to the contrast agent properties. These measurement boundary parameters may hereby particularly include parameters such as the concentration of the contrast agent upon injection, the contrast agent volume and/or the contrast agent injection rate. The adjustment of step S202 may further comprise a normalizing of the image resolution based on respective system parameters as boundary parameters, such as the projection resolution. In the specific embodiment of Fig. 2, the image sequence length is further adjusted to the contrast timing based on one or more measurement parameters as boundary parameters, such as the contrast agent volume or the like.

The adjustment of step S202 may optionally comprise selecting particular projection angles to generate a combined stack of projection images from the diagnostic images based on respective system and/or measurement boundary parameters. Hereby, some projection angles may be excluded, while others may be more preferred.

The output of step S202 in the method according to Fig. 2 corresponds to the combination result 30 comprising the diagnostic images of the time series of diagnostic images 10 adjusted based on the dataset 20. In step S203, the processing unit 200 provides the combination result 30 to the trained classifier device 300, which in the exemplary embodiment according to Fig. 2, corresponds to a neural network.

In step S301, the trained classifier device 300 receives the combination result 30 and, in step S302, classifies the combination result 30 based on the trained ground truth to generate a classification result 40.

In step S303, the trained classifier device 300 provides the thus generated classification result 40 to the processing unit 200 for further processing.

In step S204, the processing unit 200 receives the classification result 40 and determines in step S205, based on the classification result, a quantitative fluid dynamics parameter for the vasculature as represented in the time series of diagnostic images.

In step S206, the processing unit 200 then provides an information about the quantitative fluid dynamics parameter to display unit 400.

In step S401, display unit 400 receives the information about the quantitative fluid dynamics parameter and, in step S402, generates a graphical representation thereof. In step S403, the display unit 400 then displays the graphical representation of the information about the quantitative fluid dynamics parameter on a respective display device. This allows a user to review the information.

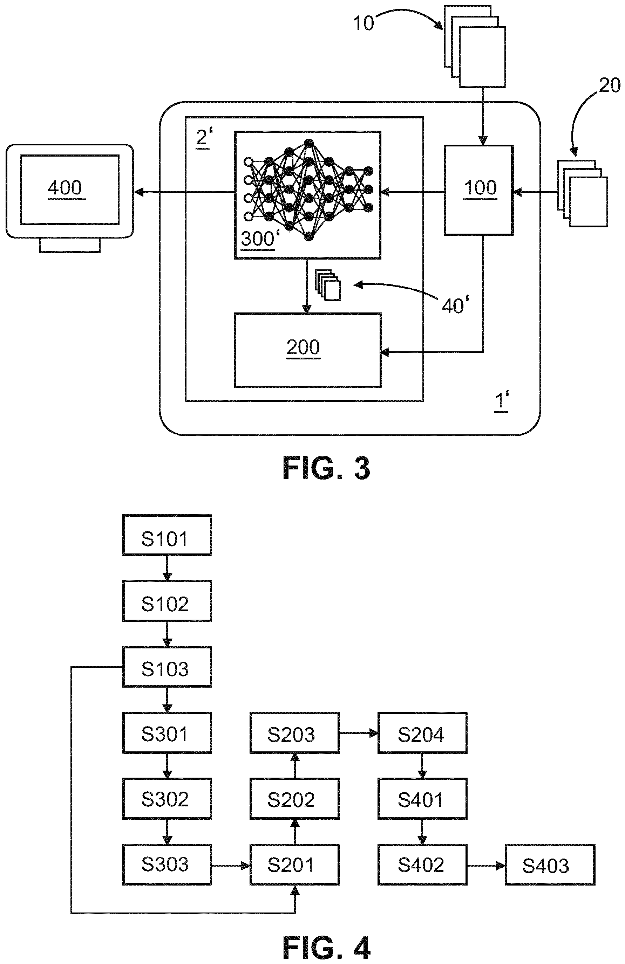

Fig. 3 represents schematically a second exemplary embodiment of an apparatus 1' for assessing a vasculature, in particular a coronary vasculature, based on at least one quantitative fluid dynamics parameter, such as a coronary flow reserve, that has been derived on the basis of a time series of diagnostic images and a set of associated boundary parameters. The apparatus 1' according to Fig. 3 largely corresponds to the apparatus according to Fig. 1. Hereby, similar components are specified using like reference numerals. That is, apparatus 1' also comprises an input unit 100 and a computation unit 2'. The computation unit of Fig. 3 also comprises a processing unit 200 and a trained classifier device 300', which, in the exemplary embodiment of Fig. 3, is implemented as a neural network comprising a plurality of nodes that are interconnected with one another.

Apparatus 1' according to Fig. 3 also comprises or is communicatively connected to a display device 400 which is configured to generate a graphical representation of the results of the assessment performed by apparatus 1'.

The procedures described in relation to the first embodiment according to Fig. 1 largely equally apply for the second embodiment according to Fig. 3. That is, input unit 100 is configured to receive a time series of diagnostic images 10 and a dataset 20 specifying at least one boundary parameter.

In the specific exemplary embodiment according to Fig. 3, the diagnostic images may particularly correspond to diagnostic images acquired using X-ray angiography upon contrast agent injection. Since the contrast agent is visible in X-ray angiography images, the diagnostic images in the time series of diagnostic images are indicative of the progression of the contrast agent through the vasculature over time. This means that, also in the exemplary embodiment of Fig. 3, the time series of diagnostic images 10 allows to track the progression of the contrast agent through the vasculature, they may be used to derive information on the fluid flow properties through the vasculature.

The dataset 20 specifying the at least one boundary parameter specifies, in the exemplary embodiment of Fig. 3, a plurality of boundary parameters which allow to adjust the diagnostic images in the time series of diagnostic images 10 as described in detail herein above.

However, contrary to the embodiment according to Fig. 1, in the exemplary embodiment according to Fig. 3, the adjusting of the time series of diagnostic images 10 in order to generate the combination result is performed after the classification by the trained classifier device 300'. Accordingly, in the specific embodiment according to Fig. 3, the trained classifier device has been trained with a ground truth relating to the time series of diagnostic images 10 as acquired rather than a ground truth relating to the combination result comprising the adjusted diagnostic images. In the specific embodiment according to Fig. 3, the time series of diagnostic images 10 is provided to the trained classifier device 300. The trained classifier device 300 classifies the time series of diagnostic images 10 and generates a respective classification result 40' comprising the classified diagnostic images.

The trained classifier device 300 provides the classification result 40' to the processing unit 200. Further, in the exemplary embodiment of Fig. 3, the input unit 100 provides a dataset 20 indicative of a plurality of boundary parameters comprising system parameters as well as measurement boundary parameters to the processing unit 200. In the specific embodiment according to Fig. 3, the dataset 20 particularly comprises an indication about the frame rate, the projection angle and the projection resolution for the time series of diagnostic images 10 as well as a contrast agent injection rate, a contrast agent volume, a contrast agent dilution, and injection pressure and an injection timing for the time series of diagnostic images 10.

In the embodiment according to Fig. 3, the processing unit 200 uses the dataset 20 indicative of the plurality of boundary parameters to adjust the classification result, in particular the classified diagnostic images. In the specific embodiment according to Fig. 3, this adjustment may comprise the same steps as specified in relation to Fig. 1. That is, the processing unit 200 may particularly perform a normalization of the frame rate and/or the image resolution, an image contrast adjustment, a sequence length adjustment and a selection of particular projection angles in order to generate the combination result 30 that is based on the classification result 40' and the dataset 20.

The processing unit then uses the combination result 30 to determine a quantitative fluid dynamics parameter for the vasculature to be assessed. A respective information or indication about the thus determined quantitative fluid dynamics parameter is then provided to display unit 400 for subsequent displaying as described in relation to Fig. 1.

Fig. 4 schematically represents a method for assessing a vasculature as performed by the apparatus 1' according to the second embodiment.

In step S101, input unit 100 receives a time series of diagnostic images 10, which, in the specific embodiment according to Fig. 4, have been acquired using X-ray angiography. Further, the input unit 100 receives, in step S102, a dataset 20 indicative of a plurality of boundary parameters associated with the time series of diagnostic images 10. In step S103, the input unit 100 provides the time series of diagnostic images 10 to the trained classifier device 300 and the dataset 20 specifying the associated set of boundary parameters to processing unit 200.

In step S301, trained classifier device 300 receives the time series of diagnostic images 10 and, in step S302, classifies the time series of diagnostic images 10 based on the trained ground truth as described herein above to generate the classification result 40' comprising a plurality of classified diagnostic images. In step S303, the trained classifier device 300 provides the classification result 40' to the processing unit 200.

In step S201, processing unit 200 receives the classification result 40' comprising the plurality of classified diagnostic images from the trained classifier device 300 and the dataset 20 from the input unit.

Subsequently, in step S202, processing unit 200 uses dataset 20 to adjust the classification result 40', particularly the plurality of classified images therein, using the dataset 20 of boundary parameters. The adjustment of the classified images in the embodiment of Fig. 4 is hereby performed in the same manner as described in relation to the embodiment of Fig. 1 and may thus comprise a normalization of the frame rate and the image resolution, an adjustment of the image contrast and the image sequence length and a selection of particular projection angles.

The processing unit then uses, in step S203, the thus generated combination result 30 based on the classification result 40' and the dataset 20 to determine the quantitative fluid dynamics parameter for the vasculature imaged in the time series of diagnostic images. In step S204, the processing unit 200 provides an information about the quantitative fluid dynamics parameter to display unit 400.

In the specific embodiment of Fig. 4, the display unit 400 receives, in step S401, the information about the quantitative fluid dynamics parameter and, in step S402, generates a graphical representation thereof. Subsequently, in step S403, the display unit 400 displays the graphical representation of the information about the quantitative fluid dynamics parameter to a user.

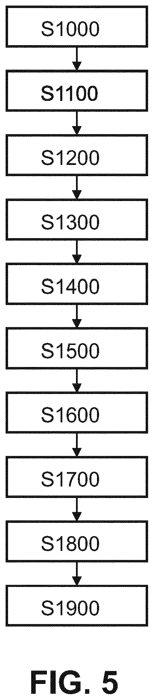

Fig. 5 schematically represents a flow chart for a method for training a classifier device based on a virtual training dataset. Said training dataset used for training the classifier device is preferably similar to an actual dataset for the use case and further comprises a ground truth for a plurality of fluid dynamics parameter values. The vasculature in the exemplary embodiment according to Fig. 5 corresponds to a coronary vasculature.

In order to train the classifier device, the embodiment according to Fig. 5 foresees, in step S 1000, that a virtual vasculature comprising a set of virtual coronary trees is specified. In step S I 100, a virtual contrast agent injection rate and a respective virtual coronary flow speed through the virtual coronary tree a specified.

Hereby, in the specific embodiment according to Fig. 5, a lumped parameter model is used to define variable flow speeds throughout the coronary tree. Particularly, in order to vary the speed of the fluid through the coronary tree, the microvascular resistance boundary conditions for the lumped parameter model are changed, such as to increase the flow speed (in case of a lower resistance) or reduce the flow speed (in case of a higher resistance).

In step S 1200, the coronary tree or coronary trees in the vasculature are combined with a motion model allowing to introduce movement of the vessels in the vasculature.

Subsequently, in step S1300, the moving vessels of the coronary tree in the vasculature are forward projected onto an empty clinical background. For each vessel in the vasculature and each coronary tree formed by the vessels, different fluid flow speeds, injection times, contrast agent concentrations and image frames per second are modelled. In some embodiments, up to 200, even up to 500 different values for the above variables may be modelled. During the modelling, different backgrounds may be selected randomly. In the specific embodiment according to Fig. 5, up to 50 or even up to 100 different coronary trees are used in order to account for variability in the coronary vasculature. Using the modelling based on these 200 to 500 different values for the variables and the 50 to 100 different coronary trees, a first training dataset is generated comprising training information about the variable factors in the training.

In step S1400, a second training dataset is generated. Hereby, the forward projected diagnostic images as also used in step S1300 are used again. However, in this case, the above-indicated variables relating to the constant injection times, contrast agent concentrations, and imaging frames per second are maintained constant. Further, the flow speeds in the coronary trees remain the same as before.

In step S1500, the second training dataset is provided to the classifier device 300. In the exemplary embodiment according to Fig. 5, the classifier device 300 corresponds to a neural network, particularly a 2.5 D encoder network architecture as shown in Fig. 7. The training of said neural network is performed using known training methods, such as back propagation, an Adam optimizer with batch normalization or the like.

The neural network having a 2.5 D encoder network architecture comprises seven input channels 51. The first seven diagnostic images of the time series of diagnostic images are provided to the seven input channels with one diagnostic image provided per channel. The corresponding ground truth output corresponds to the average fluid velocity from the lumped model.

In the specific embodiment according to Fig. 6, the network is trained for about 100 epochs in step S1600 and, subsequently, the network having the best validation error is picked. Both, a loss function cross entropy and an Adam optimizer are used.

Upon finishing training, the trained classifier device is applied, in step S1700, to the first training dataset. This application gives a plurality of fluid dynamic parameter values. The quantitative values along with the information initially input relating to the injection times, contrast agent concentrations, and imaging frames per second for the first training dataset are taken as input set for a four-dimensional linear regression in step S1800 The quantitative ground truth fluid speed is then divided by the qualitative fluid dynamic values and used as an output set of the regression in step S 1900, resulting in a respective correction factor.

Fig. 6 represents schematically a flow chart for a method for classifying an input dataset using a trained classifier device that has been trained as described herein above.

In step S2000 a time series of diagnostic images which is similar to the first training dataset is provided to the trained classifier device. In step S2100, a normalization of the data is performed by first subtracting every next frame from the previous and using a threshold on the absolute difference. This allows identifying the first frame of the contrast injection. Subsequently, starting from the first frame the next seven frames are considered. Before being provided to the trained classifier device, shutters are cropped off manually in the exemplary embodiment according to Fig. 6 and a rescaling to a fixed number of pixels is performed for the diagnostic images. Hereby, the same number of pixels as in the training data is used.

In step S2200, the resulting seven normalized frames are provided to the trained classifier device which, based thereupon, generates an output in step S2300. This output, together with the injection times, contrast agent concentrations, and imaging frames per second for the angiography data is entered into the equation resulting from the regression analysis in step S2400. Finally, in step S2500, the correction factor is multiplied with the classification result output from the trained classifier device.

Fig. 7 schematically illustrates an exemplary embodiment for a neural network that may be used as a classifier device. The neural network according to the exemplary embodiment of Fig. 7 has a 2.5 D network architecture having seven input channels 51 that are distributed onto 32 channels 52, distributed, in the next level to 64 channels 53 and 128 channels 54. The output 55 of the neural network is the average fluid speed through the vasculature.

Although in above described embodiments, the diagnostic images have been obtained using X-ray angiography, it shall be understood that in other embodiments, the diagnostic images may be obtained by other imaging methods, such as helical computed tomography or sequential computed tomography, dual energy X-ray, spectral X-ray, magnetic resonance imaging, ultrasound imaging, or the like.

Further, it shall be understood that, although in the above embodiments, the input unit and the computation unit are implemented as several separate entities, these units may also correspond to the same entity. More specifically, they may be implemented as respective modules and/or a computer program to be executed by a processing device.

Further, while in the above embodiments, the assessment has been described in particular in relation to the coronary vasculature, it shall be understood that, in other embodiments, the assessment may likewise be performed on other vascular anatomies, such as peripheral, abdominal or neurovascular. Further kinds of vascular anatomies are also foreseeable.

It may further be understood that while in the above-embodiments, the training of the classifier device has been performed on the basis of a virtually generated training dataset, the training may likewise be performed on the basis of other kinds of datasets, such as measured data that has been accordingly processed to form training dataset.

Further, it shall be understood that, although in the above embodiments, the classifier device particularly corresponds to a 2.5D encoder neural network architecture, other architectures for implementing machine learning and/or deep learning techniques may be used for the purpose of the present invention.

Other variations to the disclosed embodiments can be understood and effected by those skilled in the art in practicing the claimed invention, from a study of the drawings, the disclosure, and the appended claims.

In the claims, the word "comprising" does not exclude other elements or steps, and the indefinite article "a" or "an" does not exclude a plurality.

A single unit or device may fulfill the functions of several items recited in the claims. The mere fact that certain measures are recited in mutually different dependent claims does not indicate that a combination of these measures cannot be used to advantage.

Procedures like the generating of the combination result, the determining of the quantitative fluid dynamics parameter, the classifying of the data, the adjusting of the data, et cetera, performed by one or several units or devices can be performed by any other number of units or devices. These procedures, particularly the classifying of the data and the processing of the data in order to obtain the quantitative fluid dynamics parameter, as performed by the apparatus in accordance with the assessment method, can be implemented as program code means of a computer program and/or as dedicated hardware.

A computer program may be stored/distributed on a suitable medium, such as an optical storage medium or a solid-state medium, supplied together with or as part of other hardware, but may also be distributed in other forms, such as via the Internet or other wired or wireless telecommunication systems.

Any reference signs in the claims should not be construed as limiting the scope.

The invention relates to an apparatus for assessing a vasculature, comprising an input unit configured to receive a time series of diagnostic images of the vasculature and at least one boundary parameter associated with said time series of diagnostic images, a computation unit comprising a trained classifier device, whereby the computation unit is configured to generate a combination result based on the time series of diagnostic images and the at least one boundary parameter and determine, using the trained classifier device, a quantitative fluid dynamics parameter indicative of the fluid flow through the vasculature based on the combination result.

By means of this arrangement, an accurate, robust and simple derivation of flow-related indices which are important diagnostic indicators in the assessment of a vasculature, in particular a coronary vasculature, is achieved.

## Claims

1. An apparatus for assessing a vasculature, comprising:
an input unit configured to receive
a time series of diagnostic images of the vasculature, and
at least one boundary parameter associated with said time series of diagnostic images;
a computation unit comprising a trained classifier device, the computation unit configured to
generate a combination result based on the time series of diagnostic images and the at least one boundary parameter, and
determine, using the trained classifier device, a quantitative fluid dynamics parameter indicative of the fluid flow through the vasculature based on the combination result.

2. The apparatus according to claim 1, wherein the computation unit further comprises a processing unit, wherein
the trained classifier device is configured to
receive the time series of diagnostic images,
classify the time series of diagnostic images based on a trained ground truth to generate a classification result, and
provide the classification result to the processing unit,
wherein the processing unit is configured to
receive the classification result,
generate the combination result based on the classification result and the at least one boundary parameter, and
determine the quantitative fluid dynamics parameter based on the combination result.

3. The apparatus according to claim 1, wherein the computation unit further comprises a processing unit, wherein
the processing unit is configured to
generate the combination result based on the time series of diagnostic images and the at least one boundary parameter, and
provide the combination result to the trained classifier device, wherein
the trained classifier device is configured to
receive the combination result,
classify the combination result based on a trained ground truth to generate a classification result, and
provide the classification result to the processing unit, wherein
the processing unit is further configured to
receive the classification result based on the combination result, and
determine the quantitative fluid dynamics parameter based on the classification result.

4. The apparatus according to anyone of claims 1 to 3, wherein
the trained classifier device is trained with a ground truth for the quantitative fluid dynamics parameter, wherein
the trained classifier device is trained using a virtual time series of diagnostic images indicative of a contrast agent dynamic through the vasculature.

5. The apparatus according to claim 4, wherein the virtual time series of diagnostic images is generated by
defining at least one virtual vessel tree,
defining a virtual contrast agent injection rate, and
modelling the flow speed through the least one vessel tree based on a fluid dynamics model.

6. The apparatus according to anyone of claims 1 to 5, wherein the combination result is generated by using the at least one boundary parameter associated with said time series of diagnostic images to perform an adjustment of the time series of diagnostic images.

7. The apparatus according to claim 6, wherein the adjustment comprises one or more of:
a normalization of a frame rate,
an adjustment of an image contrast,
a normalization of an image resolution,
an adjustment of a sequence length,
a selection of projection angles.

8. The apparatus according to anyone of claims 1 to 7, wherein the at least one boundary parameter comprises at least one system parameter and/or at least one measurement boundary parameter.

9. The apparatus according to claim 8, wherein the at least one boundary parameter comprises one or more of:
a frame rate,
a projection angle,
a projection resolution,
a contrast agent injection rate,
a contrast agent volume,
a contrast agent dilution,
an injection pressure,
an injection timing.

10. The apparatus according to anyone of claims 1 to 9, wherein the computation unit comprises a processing unit, wherein the processing unit comprises a second trained classifier device.

11. A method for assessing a vasculature, comprising the steps of
receiving a time series of diagnostic images of the vasculature,
receiving at least one boundary parameter associated with said time series of diagnostic images,
generating a combination result based on the time series of diagnostic images and the at least one boundary parameter, and
determining, using a trained classifier device, a quantitative fluid dynamics parameter indicative of the fluid flow through the vasculature based on the combination result.

12. The method according to claim 11, further comprising
generating, by the trained classifier device, a classification result by receiving the time series of diagnostic images and classifying the time series of diagnostic images based on a trained ground truth,
generating the combination result based on the classification result and the at least one boundary parameter, and
determining the quantitative fluid dynamics parameter based on the combination result.

13. The method according to claim 11, further comprising
generating the combination result based on the time series of diagnostic images and the at least one boundary parameter,
classifying, by the trained classifier device, the combination result based on a trained ground truth to generate the classification result, and
determining the quantitative fluid dynamics parameter based on the classification result.

14. A computer program for controlling an apparatus according to anyone of claims 1 to 10, which, when executed by a processing device, is adapted to perform the method according to anyone of claims 11 to 13.

15. A computer-readable medium having stored thereon the computer program according to claim 14.
